(19) Europäisches Patentamt
European Patent Office
Office européen des brevets

(11) Publication number: 0 325 773 A1

# (12) EUROPEAN PATENT APPLICATION

(21) Application number: 88121407.6

(51) Int. Cl.4: C12N 15/00

(22) Date of filing: 21.12.88

(30) Priority: 28.12.87 JP 334650/87
20.04.88 JP 97114/88

(43) Date of publication of application:
02.08.89 Bulletin 89/31

(84) Designated Contracting States:
CH DE FR GB LI SE

(71) Applicant: JAPAN TOBACCO INC.
2-1 Toranomon 2-chome Minato-ku
Tokyo 105(JP)

Applicant: Samuelsson, Bengt c/o Karolinska Institutet
Box 60400
S-10401 Stockholm(SE)

Applicant: Funk, Colin c/o Karolinska Institutet
Box 60400
S-10401 Stockholm(SE)

Applicant: Radmark, Olof c/o Karolinska Institutet
Box 60400
S-10401 Stockholm(SE)

Applicant: Höög, Jan-Olov c/o Karolinska Institutet
Box 60400
S-10401 Stockholm(SE)

Applicant: Jörnvall, Hans c/o Karolinska Institutet
Box 60400
S-10401 Stockholm(SE)

(72) Inventor: Matsumoto, Takashi 1-205,
Nihontabakosangyo K.K.
Shataku-Nishishinagawa-Apt.
1-28-8,Nishishinagawa
Shinagawa-ku Tokyo(JP)
Inventor: Samuelsson, Bengt
Karolinska Institutet, Box 60 400
S-104 01 Stockholm(SE)
Inventor: Funk, Colin
Karolinska Institutet, Box 60 400
S-104 01 Stockholm(SE)
Inventor: Radmark, Olof
Karolinska Instutet, Box 60 400
S-104 01 Stockholm(SE)
Inventor: Höög, Jan-Olov
Karolinska Instutet, Box 60 400
S-104 01 Stockholm(SE)
Inventor: Jörnvall, Hans
Karolinska Instutet, Box 60 400
S-104 01 Stockholm(SE)

(74) Representative: Sajda, Wolf E., Dipl.-Phys. et al
MEISSNER, BOLTE & PARTNER
Widenmayerstrasse 48 Postfach 86 06 24
D-8000 München 86(DE)

## (54) A gene coding for human 5-lipoxygenase polypeptide.

(57) A synthetic gene which codes for human 5-lipoxygenase.

EP 0 325 773 A1

# A gene coding for human 5-lipoxygenase polypeptide

This invention relates to a gene which codes for human 5-lipoxygenase polypeptide.

5-Lipoxygenase is an enzyme which catalyzes the formation of 5-hydroperoxy-6,8,11,14-eicosatetraenoic acid (5-HPETE) from arachidonic acid, and further converts 5-HPETE into 5,6-oxido-7,9,11,14-eicosatetraenoic acid (leukotriene $A_4$). Leukotriene $A_4$ is further converted into peptide leukotriene or leukotriene $B_4$ by other enzymes. The 5-lipoxygenase thus catalyzes the first two steps in the biosynthesis of the leukotrienes, and is also involved in the formation of lipoxins, which have structures different from the leukotrienes derived from arachidonic acid.

A little has been known about the biological activity of the leukotrienes and the lipoxins. At present, however, it is known that the leukotrienes play an important role in the contraction of smooth muscle and inflammation cause. The other main biological activities include: activation of secretion from mucous membrane of bronchus, activation of insulin secretion (pancreas), activation of secretion of luteinizing hormone (brain), activation of leukocyte, activation of suppressor T-cell, reinforcement of interferon production and the like. Some of them are very useful biological activities of great possibility in terms of clinical treatments. A practical usage of human 5-lipoxygenase is considered to be applied for patients of aquired lipoxygenase disorder or the like.

It has been reported that 5-lipoxygenase can be isolated from: human leukocyte [Proc. Natl. Acad. Sci. USA vol. 82, p. 6040 (1985), vol. 82, p. 7505 (1985), vol. 83, p. 857 (1986)], porcine leukocyte (J. Biol. Chem., vol. 261, p. 7982 (1986)), murine myeloid originating mast cell [Proc. Natl. Acad. Sci. USA, vol. 83, p. 4175 (1986)], and rat basophilic leukemia cell [Prostaglandins vol. 29, p. 689 (1985)]. 5-Lipoxygenases isolated by the above methods are common in requiring calcium ion for activity manifestation. However, they show different properties in other activation factors. Further, they have molecular weight of their own such as 80 K (human), 72 K (porcine), 75 K (mouse) and 73 K (rat), implying that they are formed of different types of polypeptide.

At present, human 5-lipoxygenase can be obtained only by isolation and purification from human leukocyte. However, it is substantially impossible to permanently supply a large amount of human 5-lipoxygenase by isolation and purification from human leukocyte or organs which produce human 5-lipoxygenase.

It is, therefore, an object of the present invention to prepare a gene (DNA) coding for 5-lipoxygenase polypeptide for the mass-production of it.

According to the present invention, a gene coding for 5-lipoxygenase polypeptide which has the amino acid sequence shown in Fig. 1 is presented.

This invention can be more fully understood from the following detailed description when taken in conjunction with the accompanying drawings, in which:

Fig. 1 shows the amino acid sequence of human 5-lipoxygenase polypeptide;

Fig. 2 shows the base sequence of complementary DNA coding for human 5-lipoxygenase polypeptide;

Fig. 3 shows the base sequence of X in Fig. 2;

Fig. 4 shows the base sequence of Y in Fig. 2; and

Fig. 5 shows the result of the autoradiography.

The present inventors determined the amino acid sequence of human 5-lipoxygenase polypeptide for the first time, and synthesized a gene (complementary DNA) coding for it.

It has been identified that human 5-lipoxygenase-polypeptide has the amino acid sequence shown in Fig. 1.

To quote an example, the gene (complementary DNA) of the invention coding for human 5-lipoxygenase polypeptide has the base sequence shown in Fig. 2. This complementary DNA is composed of total 2025 base pairs from base pair number 1 to base pair number 2025 as shown in Fig. 2. Base pairs 1 to 3 correspond to a genetic code for initiating replication. The genetic code for determining the amino acid sequence of human 5-lipoxygenase begins with base pair 4 and ends with 2022. The code corresponds to 673 amino acid residue of human 5-lipoxygenase polypeptide shown in Fig. 1. In Fig. 2, base pairs 2023 to 2025 correspond to a genetic code for terminating replication.

In Fig. 2, §§§ can be any one of TGA, TAA, and TAG. X and Y indicates arbitrary base pairs corresponding to a genetic code for starting replication and a genetic code for terminating replication, respectively. The type and the number of these base pairs can be arbitrary, but they must be appropriate for linking to extranuclear genes of bacteria or the like. For example, X and Y have the base sequences shown in Fig. 3 and Fig. 4, respectively.

The complementary DNA of the present invention can be obtained from human origin complementary DNA library by an immunochemical method or a probe hybridization method. According to the immunochemical method, a complementary DNA synthesized by messenger-RNA as a template is recombinated to a gene of phenotypic bacteriophage to produce a complementary DNA library. The complementary DNA library is integrated into a bacterium to produce corresponding polypeptides. The polypeptide corresponding to human 5-lipoxygenase is searched by marking the antigen-antibody reaction, thereby obtaining the desired complementary DNA.

According to the probe hybridization method, human 5-lipoxygenase polypeptide is chemically decomposed to obtain fragments and the amino acid sequence of the fragments is determined by a chemical method. A plurality of DNA fragments anticipated from the amino acid sequence, are chemically synthesized to prepare probes. Clonal insert DNA obtained by the immunochemical method can also be used as a probe. By using these probes, DNA having the base sequence complementary to the probe can be obtained from complementary DNA library.

The complementary DNA obtained as above is inserted to a proper vector (plasmid, phage or the like) and a large amount of the complementary DNA can be produced in bacteria as hosts.

A complementary DNA library to prepare the complementary DNA of the invention can be any suitable one if it is originated in 5-lipoxygenase active human organ. For example, human placental complementary DNA library or human lung complementary DNA library can be used and these libraries are commercially available. As described above, the complementary DNA of the invention having all the necessary genetic information to prepare human 5-lipoxygenase polypeptide can be obtained.

The present invention is described by way of the following examples. The following examples are to explain a method for producing DNA having the base sequence of Fig. 2 in which X and Y have the base sequences of Figs. 3 and 4, respectively, and a method for preparing human 5-lipoxygenase polypeptide by using the produced DNA.

(a) Analysis for amino acid sequence of hydrolysate fragments of human 5-lipoxygenase polypeptide.

Human 5-lipoxygenase was isolated and purified from human leukocyte until it showed a single band in sodium dodecylsulfate polyacrylamide gel electrophoresis by an ordinary method. Four hundred µg of the purified human 5-lipoxygenase was reduced with dithiothreitol, and carboxymethylated with iodo [2-$^{14}$C] acetic acid dissolved in 0.4 M tris hydrochloric acid buffer solution (pH 8.1) containing 6 M guanidine hydrochloride and 2 mM EDTA. The obtained carboxymethylated 5-lipoxygenase polypeptide was dialized against distilled water to remove the unreacted reagents, and dissolved in 100 µℓ of 9 M deionized urea. Ammonium bicarbonate is added to the solution and the solution was diluted until the final concentration of 1 M urea and 0.1 M ammonium bicarbonate (pH 8.0). Then, 50 µg of lysine-specific protease derived from Achromobacter lyticus was immediately added to the solution. The reaction was conducted for 4 hours at 37° C. The obtained peptides were separated by reverse-phase HPLC on an Ultropac TSK ODS-120T column (a trade name), utilizing a gradient of acetonitrile (0-80 %) in 0.1 % trifluoroacetic acid.

The amino acid sequence of the peptides were analized with an Applied Biosystems 470 A gas-phase sequencer. The result obtained was indicated by the underline in the amino acid sequence of Fig. 1.

(b) Preparation of human 5-lipoxygenase antibody

Purified human 5-lipoxygenase (200 µg/900 µℓ physiological saline) was emulsified by well mixing with Freund's complete adjuvant and injected subcutaneously to 10 different locations of rabbit's back. This process was repeated every 2 weeks, 4 times altogether. Three months later, the blood was collected and antiserum was obtained.

Twenty mℓ of the antiserum was adsorbed on a column of protein A Sepharose CL-4 B (a trade name). Then 300 mℓ of 0.1 M potassium phosphate buffer solution (pH 7.1) was poured into the column followed by 0.1 M glycine hydrochloric acid buffer solution (pH 3.0) instead. Thus, eluted IgG fraction (15 mℓ) was collected. The buffer solution of the IgG fraction was replaced by 0.1 M potassium phosphate buffer solution using PD-10 column (a trade name) by an ordinary method. Sodium azide (0.05 %) was added thereto and it was saved for further usage.

(c) Screening of human lung complementary DNA library with antibody and determination of base sequence of the obtained complementary DNA.

Human lung complementary DNA library containing approximately $10^4$ bacteriophages was absorbed to Escherichia coli Y1090. Agar plates with the E. coli Y1090 were incubated at 42° C for 3.5 hours. The agar plates were then overlaid with nitrocellulose filters soaked beforehand in 10 mM isopropyl β-D-thiogalactopyranoside solution and incubated overnight at 37°C. The grown bacteriophages formed plaques on the plate, and simultaneously, polypeptide produced by the E. coli were absorbed on the nitrocellulose filters.

The filters were rinsed with TBS buffer solution (50 mM Tris hydrochloric acid buffer solution containing 150 mM sodium chloride, pH 8.0) and incubated with 10 mℓ of diluted IgG fraction [10 mℓ of TBS buffer solution containing 20 % (V/V) calf serum and 0.4 mℓ of IgG fraction obtained in example (b)], for 6 hours at room temperature. The filters were washed with TBS buffer solution, TBS buffer solution containing 0.1 % (V/V) Nonidet P-40 and TBS buffer solution in the mentioned order. The filters were put in 10 mℓ of TBS buffer solution containing 20 % (V/V) calf serum and 20 µℓ of $^{125}$I-protein A (100 µCi/mℓ) for further reaction at room temperature for 2 hours. Thereafter, the filters were washed with TBS buffer solution, 0.1 % (V/V) Nonidet P-40-TBS buffer solution, and TBS buffer solution in the order. After drying, the filters were autoradiographed at -70°C with an intensify screen.

$^{125}$I-protein A was bound to the filter corresponding to the plaque in which human 5-lipoxygenase polypeptide was present, and black dot was shown on an X-ray film. The bacteriophage of plaque which showed the black dot was isolated from the agar plate, thereby obtaining clone of human 5-lipoxygenase.

The EcoRI insert of the positive phage clone was inserted into vector M13MP18 for subcloning and the base sequence of the DNA was determined by an ordinary method (deoxy chain termination method).

The result showed that this DNA had 177 base pairs as shown by base number 1849 to 2025 of the base sequence in Fig. 2, and 220 base pairs as shown by the base sequence after terminator codon in Fig. 4, the length of total 397 base pairs. It was confirmed that the region from base number 1921 to 1953 and the region from base number 1969 to 2019 coincided with the result of analysis of polypeptide in example (a) (residues 640 to 650 and residues 656 to 672 of human 5-lipoxygenase polypeptide chain of Fig. 1), and the obtained DNA with the 397 base pairs had the genetic information identical to that of human 5-lipoxygenase.

(d) Screening of human placental complementary DNA library by probe hybridization method and determination of the base sequence of the obtained complementary DNA.

DNA fragment of the 397 base pairs obtained in example (c) was labeled with $^{32}$p to approximately $10^7$ dpm/µg by an ordinary method and used for the screening of human placental complementary DNA library.

The library containing approximately $10^4$ bacteriophages was absorbed to E. coli Y1090. Agar plates with the E. coli Y1090 were incubated overnight at 37°C. The agar plates were then overlaid with duplicate nitrocellulose filters for 1 minute each to absorb bacteriophage DNA thereon. These filters were placed on 0.5 M sodium hydroxide solution containing 1.5 M sodium chloride to denature the DNA and further placed on 0.5 M tris hydrochloric acid buffer solution (pH 8.0) containing 1.5 M sodium chloride to neutralize. After drying these filters, they were heated for 2 hours at 80°C. After the filters cooled to room temperature, the DNA bound to these filters was subsequently prehybridized in a solution containing 50 % (V/V) formamide, 5x Denhardt's solution (1x is 0.02 % Ficoll / 0.02 % polyvinylpyrrolidone / 0.02 % bovine serum albumin), 5x SSPE (1x is 0.15 M sodium chloride / 1 m MEDTA / 10 mM sodium phosphate, pH 7.4), 0.1 % sodium dodecyl sulfate, and 100 µg of denatured salmon sperm DNA per mℓ, overnight at 42°C.

The hybridization reaction was further carried out by adding $^{32}$p labeled DNA (1µg) of the 397 base pairs to the above reacted mixture for 24 hours. Thereafter, the filters were washed with 300 mℓ of 2x SSC containing 0.1 % sodium dodecyl sulfate (1x corresponds to 15 mM sod.umi citrate hydrochloric acid buffer solution (pH 7.0)) 3 times for 5 minutes each, and further with 300 mℓ of 1x SSC containing 0.1 % sodium dodecyl sulfate 2 times for 1 hour each. Finally, the filters were washed with 300 mℓ of 0.2x SSC containing 0.1 % sodium dodecyl sulfate once for 1 hour at 68°C. After drying the filters, autoradiography was performed by using the intensify screen at -70°C.

The $^{32}$p labeled DNA probe of the 397 base pairs hybridized with the bacteriophage DNA into which human 5-lipoxygense complementary DNA has been inserted. The region corresponding to plaque of bacteriophage absorbed to the nitrocellulose showed black dot on an X-ray film. The bacteriophages of the plaques showing the black dot were isolated and three clones were obtained which had insert DNA of different length. The insert DNA of each clone had approximately 1600, 2200, and 2600 base pairs, respectively (hereafter called λpℓ 9AS, λpℓ 6S, and λpℓ 5BS).

The obtained bacteriophage of each clone was inserted into vector M13MP18 for subcloning and the base sequence of each DNA was determined by an ordinary method (deoxy termination method). Clone λpℓ 5BS contains 2551 base pairs as the insert DNA and had 2073 base pairs from the initiator codon, ATG, to the terminator codon TGA in a continuous open reading frame. In this insert DNA, however, 51 base pairs from base number 1229 to 1279 shown in Fig. 2 were repeated between base pairs 1228 and 1229. The base sequences of the insert DNA for clone λpℓ 9AS and p 6S were identical to the base sequence of the insert DNA for clone λpℓ 5BS except for the repeated portion. Therefore, it was concluded that the repeated structure of the 51 base pairs which the insert DNA for clone λpℓ 5BS had was an artificial product, produced during the complementary DNA synthesis, which does not exist in the complementary DNA of the natural human 5-lipoxygenase. The correct base sequence for the complementary DNA which express the natural human 5-lipoxygenase was the one shown in Fig. 2. The complementary DNA shown in Fig. 2 was synthesized by the following steps. DNA of 422 base pairs (including the artificially produced 51 base pairs) intervening between 2 loci of the recognition base sequence for Xma1 restriction enzyme in the complementary DNA of clone λpℓ 5BS, was removed by the same enzyme mentioned above employing an ordinary method. Then, this removed DNA was replaced with DNA of 371 base pairs (not including the artificially produced pairs) intervening between 2 loci of the recognition base sequence for Xma1 restriction enzyme in the insert DNA of clone λpℓ 6S or λpℓ 9AS. The complementary DNA was synthesized by further using synthetic enzyme T4 DNA ligase. The amino acid sequence induced from the DNA base sequence of Fig. 2 was as shown in Fig. 1. The result obtained in example (a) coincided with the amino acid sequence induced from the DNA base sequence, thereby indicating that the synthesized complementary DNA corresponds to human 5-lipoxygenase.

(e) The manifestation of human 5-lipoxygenase polypeptide

In order to confirm whether bacteria express human 5-lipoxygenase polypeptide, antibody selection test was performed.

The expression vector (0.5 x $10^5$) (bacteriophage λgt 11) in which DNA having a total of 397 base pairs were inserted, which consisted of 177 base paires from base number 1849 to 2025 of the complementary DNA shown in Fig. 2 and 220 base pairs from those after the terminator codon shown in Fig.. 4, and the same number of bacteriophage λgt 11 without insert DNA were absorbed to E. coli Y1090. Agar plates with those E. coli Y1090 were incubated at 42° C for 3.5 hours. Nitrocellulose filters soaked beforehand in 10 mM isopropyl β-D-thiogalactopyranoside aqueous solution were placed on the agar plates and further incubated overnight at 37° C.

The grown bacteriophages formed plaques, and simultaneously polypeptide produced by the E. coli were absorbed to the nitrocellulose filters. The filters were rinsed with TBS buffer solution, and incubated with 10 mℓ of diluted IgG fraction for 6 hours at room temperature. Thereafter, the filters were washed with TBS buffer solution, TBS buffer solution containing 0.1 % (V/V) Nonidet P-40, and TBS buffer solution in the mentioned order. It was, then, shaked in 2 mℓ of 0.1 M glycine hydrochloric acid buffer solution (pH 2.6) containing 0.15 M sodium chloride for 15 minutes at 20° C, thereby absorbed antibody was extracted.

Two mℓ of 1 M tris hydrochloric acid buffer solution (pH 8.0) was added to the extract solution to neutralize, and further 6 mℓ of TBS buffer solution containing 20 % (V/V) calf serum and 0.05 % sodium azide was added thereto. The resultant solution was used for blot test.

One μg, 0.5 μg, and 0.25 μg of the purified human 5-lipoxygenase were absorbed to a nitrocellulose filter respectively. After drying, it was washed with TBS buffer solution and soaked in TBS buffer solution containing 20 % (V/V) calf serum for 30 minutes. This filter was further soaked in the above-mentioned antibody extract solution (approximately 10 mℓ) and the antigen-antibody reaction was carried out overnight. The filter was washed with TBS buffer solution, TBS buffer solution containing 0.1 % (V/V) Nonidet P-40, TBS buffer solution in the mentioned order. The filter was, then, put in 10 m of TBS buffer solution containing 20 % (V/V) calf serum and 20 μℓ $^{125}$I-protein A (100 μ Ci/mℓ) for the further reaction at room temperature for 2 hours. After that, the filter was washed again with TBS buffer solution, TBS buffer solution containing 0.1 % (V/V) Nonidet P-40, and TBS buffer solution in the mentioned order. After drying, the autoradiography was performed using intensifying screen at -70° C.

As shown in Fig. 5, the antibody bound to polypeptide produced by E. coli transfected with bacteriophage λgt11 having the 397 base pairs as insert DNA, reacted with human 5-lipoxygenase polypeptide, thereby showing a black dot of a X-ray film as the indication of dose response (Fig. 5-(a)). On the other hand, the polypeptide produced by E. coli transfected with bacteriophage λgt 11 without insert DNA did not show a black dot on the X-ray film. The result shows that bacteriophage λgt 11 having insertion DNA of the 397 base pairs caused E. coli to produce part of human 5-lipoxygenase polypeptide.

As has been described above, according to the present invention, a gene coding for human 5-lipoxygenase is produced, thereby human 5-lipoxygenase can be mass-produced.

## Claims

1. A synthetic gene which codes for human 5-lipoxygenase having an amino acid sequence as shown in Fig. 1.

2. A gene having a base sequence as shown in Fig. 2 where §§§ is TGA, TAA, or TAG, X has a base sequence as shown in Fig. 3 and Y has a base sequence as shown in Fig. 4.

Pro Ser Tyr Thr Val Thr Val Ala Thr Gly Ser Gln Trp Phe Ala Gly Thr Asp Asp Tyr Ile Tyr Leu Ser Leu Val Gly Ser Ala 29
Gly Cys Ser Glu Lys His Leu Leu Asp Lys Pro Phe Tyr Asn Asp Phe Glu Arg Gly Ala Val Asp Ser Tyr Asp Val Thr Val Asp Glu 59
Glu Leu Gly Glu Ile Gln Leu Val Arg Ile Glu Lys Arg Lys Tyr Trp Leu Asn Asp Asp Trp Tyr Leu Lys Tyr Ile Thr Leu Lys Thr 89
Pro His Gly Asp Tyr Ile Glu Phe Pro Cys Tyr Arg Trp Ile Thr Gly Asp Val Glu Val Val Leu Arg Asp Gly Arg Ala Lys Leu Ala 119
Arg Asp Asp Gln Ile His Ile Leu Lys Gln His Arg Arg Lys Glu Leu Glu Thr Arg Gln Lys Gln Tyr Arg Trp Met Glu Trp Asn Pro 169
Gly Phe Pro Leu Ser Ile Asp Ala Lys Cys His Lys Asp Leu Pro Arg Asp Ile Gln Phe Asp Ser Glu Lys Gly Val Asp Phe Val Leu 179
Asn Tyr Ser Lys Ala Met Glu Asn Leu Phe Ile Asn Arg Phe Met His Met Phe Gln Ser Ser Trp Asn Asp Phe Ala Asp Phe Glu Lys 209
Ile Phe Val Lys Ile Ser Asn Thr Ile Ser Glu Arg Val Met Asn His Trp Gln Glu Asp Leu Met Phe Gly Try Gln Phe Leu Asn Gly 239
Cys Asn Pro Val Leu Ile Arg Arg Cys Thr Glu Leu Pro Glu Lys Leu Pro Val Thr Thr Glu Met Val Glu Cys Ser Leu Glu Arg Gln 269
Leu Ser Leu Glu Gln Glu Val Gln Gln Gly Asn Ile Phe Ile Val Asp Phe Glu Leu Leu Asp Gly Ile Asp Ala Asn Lys Thr Asp Pro 299
Cys Thr Leu Gln Phe Leu Ala Ala Pro Ile Cys Leu Leu Tyr Lys Asn Leu Ala Asn Lys Ile Val Pro Ile Ala Ile Gln Leu Asn Gln 329
Ile Pro Gly Asp Glu Asn Pro Ile Phe Leu Pro Ser Asp Ala Lys Tyr Asp Trp Leu Leu Ala Lys Ile Trp Val Arg Ser Ser Asp Phe 359
His Val His Gln Thr Ile Thr His Leu Leu Arg Thr His Leu Val Ser Glu Val Phe Gly Ile Ala Met Tyr Arg Gln Leu Pro Ala Val 389
His Pro Ile Phe Lys Leu Leu Val Ala His Val Arg Phe Thr Ile Ala Ile Asn Thr Lys Ala Arg Glu Gln Leu Ile Cys Glu Cys Gly 419
Leu Phe Asp Lys Ala Asn Ala Thr Gly Gly Gly Gly His Val Gln Met Val Gln Arg Ala Met Lys Asp Leu Thr Tyr Ala Ser Leu Cys 449
Phe Pro Glu Ala Ile Lys Ala Arg Gly Met Glu Ser Lys Glu Asp Ile Pro Tyr Tyr Phe Tyr Arg Asp Asp Gly Leu Leu Val Trp Glu 479
Ala Ile Arg Thr Phe Thr Ala Glu Val Val Asp Ile Tyr Tyr Glu Gly Asp Gln Val Val Glu Glu Asp Pro Glu Leu Gln Asp Phe Val 509
Asn Asp Val Tyr Val Tyr Gly Met Arg Gly Arg Lys Ser Ser Gly Phe Pro Lys Ser Val Lys Ser Arg Glu Gln Leu Ser Glu Tyr Leu 539
Thr Val Val Ile Phe Thr Ala Ser Ala Gln His Ala Ala Val Asn Phe Gly Gln Tyr Asp Trp Cys Ser Trp Ile Pro Asn Ala Pro Pro 569
Thr Met Arg Ala Pro Pro Pro Thr Ala Lys Gly Val Val Thr Ile Glu Gln Ile Val Asp Thr Leu Pro Asp Arg Gly Arg Ser Cys Trp 599
His Leu Gly Ala Val Trp Ala Leu Ser Gln Phe Gln Glu Asn Glu Leu Phe Leu Gly Met Tyr Pro Glu Glu His Phe Ile Glu Lys Pro 629
Val Lys Glu Ala Met Ala Arg Phe Arg Lys Asn Leu Glu Ala Ile Val Ser Val Ile Ala Glu Arg Asn Lys Lys Lys Gln Leu Pro Tyr 659
Tyr Tyr Leu Ser Pro Asp Arg Ile Pro Asn Ser Val Ala Ile

FIG. 1

X

```
ATG CCC TCC TAC ACG GTC ACC GTG GCC ACT GGC AGC CAG TGG TTC GCC GGC ACT GAC GAC TAC ATC TAC CTC AGC CTC GTG GGC TCG GCG   90
GGC TGC AGC GAG AAG CAC CTG CTG GAC AAG CCC TTC TAC AAC GAC TTC GAG CGT GGC GCG GTG GAT TCA TAC GAC GTG ACT GTG GAC GAG  180
GAA CTG GGC GAG ATC CAG CTG GTC AGA ATC GAG AAG CGC AAG TAC TGG CTG AAT GAC GAC TGG TAC CTG AAG TAC ATC ACG CTG AAG ACG  270
CCC CAC GGG GAC TAC ATC GAG TTC CCC TGC TAC CGC TGG ATC ACC GGC GAT GTC GAG GTT GTC CTG AGG GAT GGA CGC GCA AAG TTG GCC  360
CGA GAT GAC CAA ATT CAC ATT CTC AAG CAA CAC CGA CGT AAA GAA CTG GAA ACA CGG CAA AAA CAA TAT CGA TGG ATG GAG TGG AAC CCT  450
GGC TTC CCC TTG AGC ATC GAT GCC AAA TGC CAC AAG GAT TTA CCC CGT GAT ATC CAG TTT GAT AGT GAA AAA GGA GTG GAC TTT GTT CTG  540
AAT TAC TCC AAA GCG ATG GAG AAC CTG TTC ATC AAC CGC TTC ATG CAC ATG TTC CAG TCT TCT TGG AAT GAC TTC GCC GAC TTT GAG AAA  630
ATC TTT GTC AAG ATC AGC AAC ACT ATT TCT GAG CGG GTC ATG AAT CAC TGG CAG GAA GAC CTG ATG TTT GGC TAC CAG TTC CTG AAT GGC  720
TGC AAC CCT GTG TTG ATC CGG CGC TGC ACA GAG CTG CCC GAG AAG CTC CCG GTG ACC ACG GAG ATG GTA GAG TGC AGC CTG GAG CGG CAG  810
CTC AGC TTG GAG CAG GAG GTC CAG CAA GGG AAC ATT TTC ATC GTG GAC TTT GAG CTG CTG GAT GGC ATC GAT GCC AAC AAA ACA GAC CCC  900
TGC ACA CTC CAG TTC CTG GCC GCT CCC ATC TGC TTG CTG TAT AAG AAC CTG GCC AAC AAG ATT GTC CCC ATT GCC ATC CAG CTC AAC CAA  990
ATC CCG GGA GAT GAG AAC CCT ATT TTC CTC CCT TCG GAT GCA AAA TAC GAC TGG CTT TTG GCC AAA ATC TGG GTG CGT TCC AGT GAC TTC 1080
CAC GTC CAC CAG ACC ATC ACC CAC CTT CTG CGA ACA CAT CTG GTG TCT GAG GTT TTT GGC ATT GCA ATG TAC CGC CAG CTG CCT GCT GTG 1170
CAC CCC ATT TTC AAG CTG CTG GTG GCA CAC GTG AGA TTC ACC ATT GCA ATC AAC ACC AAG GCC CGT GAG CAG CTC ATC TGC GAG TGT GGC 1260
CTC TTT GAC AAG GCC AAC GCC ACA GGG GGC GGT GGG CAC GTG CAG ATG GTG CAG AGG GCC ATG AAG GAC CTG ACC TAT GCC TCC CTG TGC 1350
TTT CCC GAG GCC ATC AAG GCC CGG GGC ATG GAG AGC AAA GAA GAC ATC CCC TAC TAC TTC TAC CGG GAC GAC GGG CTC CTG GTG TGG GAA 1440
GCC ATC AGG ACG TTC ACG GCC GAG GTG GTA GAC ATC TAC TAC GAG GGC GAC CAG GTG GTG GAG GAG GAC CCG GAG CTG CAG GAC TTC GTG 1530
AAC GAT GTC TAC GTG TAC GGC ATG CGG GGC CGC AAG TCC TCA GGC TTC CCC AAG TCG GTC AAG AGC CGG GAG CAG CTG TCG GAG TAC CTG 1620
ACC GTG GTG ATC TTC ACC GCC TCC GCC CAG CAC GCC GCG GTC AAC TTC GGC CAG TAC GAC TGG TGC TCC TGG ATC CCC AAT GCG CCC CCA 1710
ACC ATG CGA GCC CCG CCA CCG ACT GCC AAG GGC GTG GTG ACC ATT GAG CAG ATC GTG GAC ACG CTG CCC GAC CGC GGC CGC TCC TGC TGG 1800
CAT CTG GGT GCA GTG TGG GCG CTG AGC CAG TTC CAG GAA AAC GAG CTG TTC CTG GGC ATG TAC CCA GAA GAG CAT TTT ATC GAG AAG CCT 1890
GTG AAG GAA GCC ATG GCC CGA TTC CGC AAG AAC CTC GAG GCC ATT GTC AGC GTG ATT GCT GAG CGC AAC AAG AAG AAG CAG CTG CCA TAT 1980
TAC TAC TTG TCC CCA GAC CGG ATT CCG AAC AGT GTG GCC ATC §§§ Y
```

FIG. 2

-34 -1

GGGCCCGGCGCTCGCTGCTCCCGCGGCCCGCGCC

FIG. 3

| | |
|---|---|
| GCACACTGCCAGTCTCACTGTGGGAAGGCCAGCTGCCCCAGCCAGATGGACTCCAGCCT | 2084<br>673 |
| GCCTGGCAGGTGTCTGGCCAGGCCTCTTGGCAGTCACATCTCTTCCTCCGAGGCCAGTACCTTTCCATTTATTCTTTGATCTTCAGGGAACTGCATAGATTGATCAAAGTGTAAACACC | 2203 |
| ATAGGGACCCATTCTACACAGAGCAGGACTGCACAGCGTCCTGTCCACACCCAGCTCAGCATTTCCACACCAAGCAGCAACAGCAAATCACGACCACTGATAGATGTCTATTCTTGTTG | 2322 |
| GAGACATGGGATGATTATTTTCTGTTCTATTTGTGCTTAGTCCAATTCCTTGCACATAGTAGGTACCCAATTCAATTACTATTGAATGAATTAAGAATTGGTTGCCATAAAAATAAATC | 2441 |
| AGTTCATTTAAAAAAAAAAAAAAAAA | 2466 |

FIG. 4

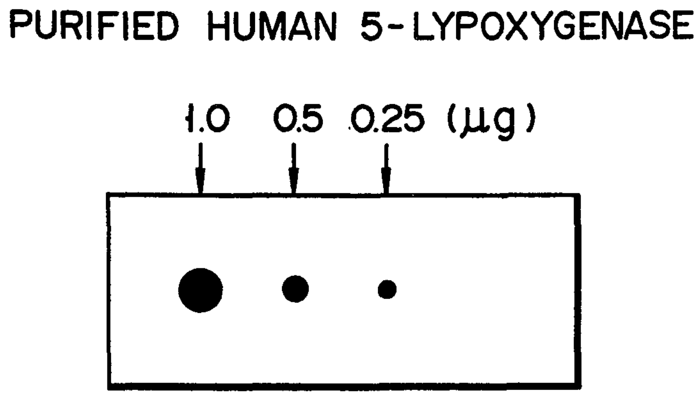


F I G. 5A

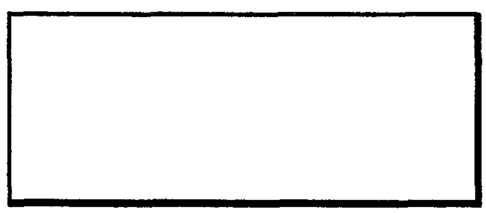

F I G. 5B

European Patent Office

# EUROPEAN SEARCH REPORT

Application Number

EP 88 12 1407

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | PROC. NATL. ACAD. SCI. USA, vol. 82, September 1985, pages 6040-6044; C.A. ROUZER et al.: "On the nature of the 5-lipoxygenase reaction in human leukocytes: Enzyme purification and requirement for multiple stimulatory factors" --- | | C 12 N 15/00 |
| P,X | PROCEEDINGS OF THE NATIONAL ACADEMY OF SCIENCES USA, vol. 85, no. 1, January 1988, pages 26-30, Washington, DC, US; T. MATSUMOTO et al.: "Molecular cloning and amino acid sequence of human 5-lipoxygenase" * Whole article * --- | 1,2 | |
| P,X | PROCEEDINGS OF THE NATIONAL ACADEMY OF SCIENCES USA, vol. 85, no. 2, January 1988, pages 416-420, Washington, DC, US; R.A.F. DIXON et al.: "Cloning of the cDNA for human 5-lipoxygenase" * Whole article * --- | 1,2 | TECHNICAL FIELDS SEARCHED (Int. Cl.4) |
| P,X | THE JOURNAL OF BIOLOGICAL CHEMISTRY, vol. 263, no. 21, 25th July 1988, pages 10135-10140, The American Society for Biochemistry and Molecular Biology, Inc., US; C.A. ROUZER et al.: "Characterization of cloned human leukocyte 5-lipoxygenase expressed in mammalian cells" * Whole article * ----- | 1,2 | C 12 N |

The present search report has been drawn up for all claims

| Place of search | Date of completion of the search | Examiner |
|---|---|---|
| THE HAGUE | 16-05-1989 | PULAZZINI A.F.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document